# EUROPEAN PATENT SPECIFICATION

(11) **EP 2 773 605 B1**
(45) Date of publication and mention of the grant of the patent: **10.12.2025**
(21) Application number: 12845281.0
(22) Date of filing: 02.11.2012
(51) Int. Cl.: C07C 17/20, C07C 17/25, C07C 17/087, C07C 19/10, C07C 21/18, B01J 23/26, B01J 27/128, B01J 37/26

(54) **PROCESS FOR PRODUCING 2,3,3,3-TETRAFLUOROPROPENE**
VERFAHREN ZUR HERSTELLUNG VON 2,3,3,3-TETRAFLUORPROPEN
PROCÉDÉ DE PRODUCTION DE 2,3,3,3-TÉTRAFLUOROPROPÈNE

(30) Priority: 04.11.2011 US 201161555682 P
(43) Date of publication of application: 10.09.2014
(60) Divisional application: 24211556.6
(73) Proprietor: Honeywell International, Inc., Morristown, NJ 07962 (US)
(72) Inventor: Bektesevic, Selma, Morris Plains, NJ 07950 (US); Merkel, Daniel C., Morris Plains, NJ 07950 (US); Tung, Hsueh Sung, Morris Plains, NJ 07950 (US); Wang, Haiyou, Morris Plains, NJ 07950 (US); Nappa, Mario Joseph, Wilmington, DE 19801 (US); Sun, Xuehui, Wilmington, DE 19801 (US)
(74) Representative: Stepney, Gregory John
(86) International application number: PCT/US2012/063320
(87) International publication number: WO 2013/067350

(56) References cited:
- WO-A1-2010/123148
- WO-A1-2011/087825
- WO-A1-2011/110889
- US-A1- 2009 030 244
- US-A1- 2009 240 090
- US-A1- 2010 036 179
- US-A1- 2011 105 807
- US-A1- 2011 105 807

## Description

### FIELD OF THE INVENTION

The present invention relates to a process for preparing fluorinated organic compounds, more particularly to a process for preparing fluorinated olefins, and even more particularly to a process for producing 2,3,3,3-tetrafluoropropene (HFO-1234yf).

### BACKGROUND OF THE INVENTION

Hydrofluoroolefins (HFOs), such as tetrafluoropropenes (including 2,3,3,3- tetrafluoropropene (HFO-1234yf)), are now known to be effective refrigerants, fire extinguishants, heat transfer media, propellants, foaming agents, blowing agents, gaseous dielectrics, sterilant carriers, polymerization media, particulate removal fluids, carrier fluids, buffing abrasive agents, displacement drying agents and power cycle working fluids. Unlike chlorofluorocarbons (CFCs) and hydrochlorofluorocarbons (HCFCs), both of which potentially damage the Earth's ozone layer, HFOs do not contain chlorine and, thus, pose no threat to the ozone layer. HFO-1234yf has also been shown to be a low global warming compound with low toxicity and, hence, can meet increasingly stringent requirements for refrigerants in mobile air conditioning. Accordingly, compositions containing HFO-1234yf are among the materials being developed for use in many of the aforementioned applications.

Several methods of preparing HFOs are known. For example, U.S. Pat. No. 4,900,874 (Ihara et al) describes a method of making fluorine containing olefins by contacting hydrogen gas with fluorinated alcohols. Although this appears to be a relatively high-yield process, commercial scale handling of hydrogen gas at high temperature is hazardous. Also, the cost of commercially producing hydrogen gas, such as building an onsite hydrogen plant, is economically costly.

U.S. Pat. No. 2,93 1,840 (Marquis) describes a method of making fluorine containing olefins by pyrolysis of methyl chloride and tetrafluoroethylene or chlorodifluoromethane. This process is a relatively low yield process and a very large percentage of the organic starting material is converted to unwanted and/or unimportant byproducts, including a sizeable amount of carbon black which tends to deactivate the catalyst used in the process.

The preparation of HFO-1234yf from trifluoroacetylacetone and sulfur tetrafluoride has been described (See Banks, et al., Journal of Fluorine Chemistry, Vol. 82, Iss. 2, p. 171-174 (1997)). Also, U.S. Pat. No. 5,162,594 (Krespan) discloses a process wherein tetrafluoroethylene is reacted with another fluorinated ethylene in the liquid phase to produce a polyfluoroolefin product. Also, US20110105807A1 discloses a process for the manufacture of HFO-1234yf from TCP in three integrated steps that include hydrofluorination of TCP (tetrachloropropene) to HCFC-1233xf in the vapor phase followed by hydrofluorination of HCFC-1233xf to HCFC-244bb in the liquid phase which is then followed by dehydrochlorination in liquid or vapor phase to produce HFO-1234yf.

However, there remains a need for an economic means of producing hydrofluoroolefins, such as HFO-1234yf. The present invention satisfies this need among others.

### SUMMARY OF INVENTION

The present invention relates, in part, to one or more process steps for improving the reaction efficiency used for the production of HFOs, such as 2,3,3,3-tetrafluoropropene (1234yf).

In one aspect, the present invention relates to a process for preparing 2-chloro-3,3,3-trifluoropropene comprising: vaporizing a fluorination agent and a composition comprising at least one compound of formula I

CX₂=CCl-CH₂X (I)

wherein X is independently selected from F, CI, Br, and I, provided that at least one X is not fluorine, wherein the at least one compound of formula I comprises 1,1,2,3-tetrachloropropene; and one or more organic co-feed compounds, wherein said one or more organic co-feed compounds are selected from the group consisting of dichlorodifluoropropene (1232), 1,2-dichloro-3,3,3-trifluoropropene (1223xd), 2-chloro-3,3,3-trifluoropropene (1233xf), 3,3,3,2-tetrafluoroprop-1-ene (1234yf), 2-chloro-1,1,1,2-tetrafluoropropane (244bb), 1,1,1,2,2-pentafluoropropane (HFC-245cb), 1,1,1,2,3-pentafluoropropane (245eb), tetrachlorofluoropropane (241), trichlorodifluoropropane (242), dichlorotrifluoropropane (243) and combinations thereof, said one or more organic co-feed compounds being present in an amount ranging from 1 to 50 wt %, and preferably from 3 to 30 wt% and more preferably from 5 to 15 wt%; and contacting the vaporized compound of formula I and the vaporized fluorinating agent in the vapor phase in the presence of a fluorination catalyst, to produce a final composition comprising 2-chloro-3,3,3-trifluoropropene.

The organic co-feed compound improves the foregoing process, particularly by decreasing starting reagent oligomerization/polymerization and/or reducing catalyst deactivation over the course of the process. In one embodiment, the organic co-feed compound has a boiling point that is lower than the compound of Formula I.

The effective amount of the co-feed compound is between 1 to 50 wt%, between 3 to 30 wt%, or between 5 to 15 wt%, each based on the total amount of organic feed provided to the reaction.

Vapor phase catalysts used for such a reaction include, but are not limited to, a chromium oxide, a chromium hydroxide, a chromium halide, a chromium oxyhalide, an aluminum oxide, an aluminum hydroxide, an aluminum halide, an aluminum oxyhalide, a cobalt oxide, a cobalt hydroxide, a cobalt halide, a cobalt oxyhalide, a manganese oxide, a manganese hydroxide, a manganese halide, a manganese oxyhalide, a nickel oxide, a nickel hydroxide, a nickel halide, a nickel oxyhalide, an iron oxide, an iron hydroxide, an iron halide, an iron oxyhalide, inorganic salts thereof, fluorinated derivatives thereof and combinations thereof. In certain embodiments, the catalyst includes a chromium oxide, such as, but not limited to Cr₂O₃.

The step of contacting the starting composition with a fluorinating agent also may occur in the presence of one or more stabilizers. Such stabilizers may include amine-based stabilizer, of which one or more of the following may be included: p-tap (4-tert-amylphenol), methoxy-hydroquinone, 4-methoxyphenol (HQMME), triethylamine, diisopropyl amine, butylated hydroxy anisole (BHA), and thymol.

In even further aspects, the present invention relates to a process for preparing 2,3,3,3 - tetrafluoroprop-1-ene by
a. vaporizing a composition comprising at least one compound of formula I

   CX₂=CCl-CH₂X (I)

   wherein X is independently selected from F, CI, Br, and I, provided that at least one X is not fluorine, wherein the at least one compound of formula I comprises 1,1,2,3-tetrachloropropene; a first fluorinating agent and one or more organic co-feed compounds, wherein said one or more organic co-feed compounds is selected from the group consisting of dichlorodifluoropropene (1232), 1,2-dichloro-3,3,3-trifluoropropene (1223xd), 2-chloro-3,3,3-trifluoropropene (1233xf), 3,3,3,2-tetrafluoroprop-1-ene (1234yf), 2-chloro-1,1,1,2-tetrafluoropropane (244bb), 1,1,1,2,2-pentafluoropropane (HFC-245cb), 1,1,1,2,3-pentafluoropropane (245eb), tetrachlorofluoropropane (241), trichlorodifluoropropane (242), dichlorotrifluoropropane (243) and combinations thereof, said at least one or more organic co-feed compounds being present in an amount ranging from 1 to 50 wt %, preferably from 3 to 30 wt % and more preferably from 5 to 15 wt %;
b. contacting said vaporized compound of formula I and said vaporized first fluorinating agent with a fluorination catalyst to produce a first intermediate composition comprising 2-chloro-3,3,3-trifluoropropene;
c. contacting said first intermediate composition with a second fluorinating agent to produce a second intermediate composition comprising 2-chloro-1,1,1,2-tetrafluoropropane; and
d. dehydrochlorinating at least a portion of the 2-chloro-1,1,1,2-tetrafluoropropane to produce a reaction product including 2,3,3,3-tetrafluoroprop-1-ene.

In even further aspects, the present invention relates to a multistep process for preparing 2,3,3,3-tetrafluoropropene (1234yf) comprising:
a.) vaporizing a starting composition comprising at least one compound of formula I

   CX₂=CCl-CH₂X (I)

   wherein X is independently selected from F, Cl, Br, and I, provided that at least one X is not fluorine, wherein the at least one compound of formula I comprises 1,1,2,3-tetrachloropropene; and a fluorinating agent and one or more organic co-feed compounds selected from the group consisting of dichlorodifluoropropene (1232), 1,2-dichloro-3,3,3-trifluoropropene (1223xd), 2-chloro-3,3,3-trifluoropropene (1233xf), 3.3.3 3,3,3,2-tetrafluoroprop-1-ene (1234yf), 2-chloro-1,1,1,2-tetrafluoropropane (244bb), 1,1,1,2,2-pentafluoropropane (HFC-245cb), 1,1,1,2,3-pentafluoropropane (245eb), tetrachlorofluoropropane (241), trichlorodifluoropropane (242), dichlorotrifluoropropane (243) and combinations thereof, said one or more organic co-feed compounds being present in an amount ranging from 1 to 50 wt %, preferably from 3 to 30 wt % and more preferably from 5 to 15 wt%, and contacting the vaporized compound of formula I and fluorinating agent with a fluorination catalyst to produce a first intermediate composition comprising 2-chloro-3,3,3-trifluoropropene (1233xf), HCl and one or more organic co-feed compounds, said one or more organic co-feed compounds being other than the compound of formula I;
b.) separating said HCl, said 2-chloro-3,3,3-trifluoropropene (1233xf), and said one or more organic co-feed compounds from said first intermediate composition;
c.) recycling an effective amount of said separated one or more organic co-feed compounds to said first vapor phase reactor;
d.) contacting, in a liquid phase reactor, said separated 2-chloro-3,3,3-trifluoropropene (1233xf) with a fluorinating agent to produce a second intermediate composition comprising 2-chloro-1,1,1,2-tetrafluoropropane (244bb); and
e.) dehydrochlorinating, in a second vapor phase reactor, at least a portion of said 2-chloro-1,1,1,2-tetrafluoropropane (244bb) to produce a reaction product comprising 2,3,3,3-tetrafluoropropene.

Additional embodiments and advantages to the present invention will be readily apparent to one of skill in the art, based on the disclosure provided herein.

### BRIEF DESCRIPTION OF THE FIGURES

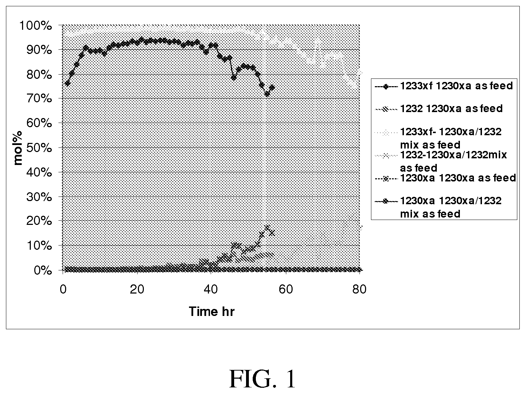

Figure 1 provides a comparative illustration of catalytic deactivation during fluorination of 1230xa to form 1233xf in the absence or presence of a 1232xf co-feed.

### DETAILED DESCRIPTION OF THE INVENTION

According to one embodiment, the present invention includes a manufacturing process for making 2,3,3,3-tetrafluoroprop-1-ene using a starting material according to formula I :

CX₂=CC1-CH₂X (Formula I)

wherein X is independently selected from F, Cl, Br, and I, provided that at least one X is not fluorine. In certain embodiments, the compound(s) of Formula I contains at least one chlorine, a majority of the Xs as chlorine, or all Xs as chlorine. The compound(s) of formula I comprises 1,1,2,3-tetrachloropropene (1230xa). Processes applicable to the present invention include, without limitation, integrated multistep processes as described in US Patent No. 8,084,653 and US Published Patent Application 2009/0240090.

The method generally includes at least three reaction steps. In the first step, a starting composition of Formula I (comprising 1,1,2,3-tetrachloropropene) is reacted with anhydrous HF in a first vapor phase reactor (fluorination reactor) to produce a mixture of 2-chloro-3,3,3-trifluoropropene (1233xf) and HCl. The reaction occurs in the vapor phase in the presence of a fluorination catalyst, such as, but not limited to, a fluorinated chromium oxide. The catalyst may (or may not) have to be activated with anhydrous hydrogen fluoride HF (hydrogen fluoride gas) before use depending on the state of the catalyst. In one embodiment, there is no oxygen-containing agent or gas feed, e.g. air, pure oxygen, or diluted oxygen gas, such as an oxygen/inert gas (e.g. nitrogen), to the first vapor phase reactor.

While fluorinated chromium oxides are disclosed as the fluorination catalyst, the present invention is not limited to this embodiment. Any fluorination catalysts known in the art may be used in this process. Suitable catalysts include, but are not limited to chromium, aluminum, cobalt, manganese, nickel and iron oxides, hydroxides, halides, oxyhalides, inorganic salts thereof and their mixtures and any one of which may be optionally fluorinated. Combinations of catalysts suitable for the present invention nonexclusively include Cr₂O₃, FeCl₃/C, Cr₂O₃/Al₂O₃, Cr₂O₃/AlF₃, Cr₂O₃/carbon, CoCl₂/Cr₂O₃/Al₂O₃, NiCl₂/Cr₂O₃/Al₂O₃, CoCl₂/AlF₃, NiCl₂/AlF₃ and mixtures thereof. Chromium oxide/aluminum oxide catalysts are described in U.S. Pat. No. 5,155,082. Chromium (III) oxides such as crystalline chromium oxide or amorphous chromium oxide are preferred with amorphous chromium oxide being most preferred. Chromium oxide (Cr 0 3) is a commercially available material which may be purchased in a variety of particle sizes. Fluorination catalysts having a purity of at least 98% are preferred. The fluorination catalyst is present in an excess but in at least an amount sufficient to drive the reaction.

The compound of formula I is also provided with at least one co-feed organic compound. The compound preferably, though not exclusively, has a boiling point that is lower than the compound of Formula I, particularly 1230xa. Non-limiting examples of such halocarbons and haloolefins include one or any combination of 2,3-dichloro-3,3-difluoropropene (1232xf), 1,2-dichloro-3,3,3-trifluoropropene (1223xd), 2-chloro-3,3,3-trifluoropropene (1233xf), 2-chloro-1,1,1,2-tetrafluoropropane (244bb), 1,1,1,2,2-pentafluoropropane (HFC-245cb). In one embodiment, the starting composition of Formula I (e.g. 1230xa), HF, and the at least one organic co-feed compounds are in the liquid phase; these are passed through a vaporizer and the resultant vapor phase materials being fed to the first vapor phase reactor.

The amount of co-feed compounds or an "effective amount," as used herein, relates to an amount that may be used to improve the conversion of a compound of formula I, particularly 1230xa, to 1233xf. The effective amount of co-feed organic compound measurably reduces the occurrence of oligomerization/polymerization of a compound of formula I during steam vaporization or during the fluorination reaction. Similarly, an effective amount may also, or independently, include an amount of the organic co-feed that results in a measurable reduction of catalyst deactivation, particularly deactivation caused by starting reagent oligomerization/polymerization. The percentage of co-feed organic(s) in total organic feed can be ranged from 1 to 50 wt%, from 3 to 30 wt%, or from 5 to 15 wt%, each based upon the total weight of organic reagents used. While not intending to be bound by theory, it is believed that co-feeding at least an organic compound with a lower boiling point than HCO-1230xa can facilitate the vaporization of HCO-1230xa and avoid/reduce the formation of HCO-1230xa oligomers, resulting in an improved catalyst life. In one embodiment, the co-feed compounds are provided as fresh feeds in effective amounts, i.e., they are not obtained from recycle streams derived from the multistep process. In another embodiment, the co-feed compounds are present in one or more recycle streams derived from the multistep process as described e.g. in US Patent No. 8,084,653 and US Published Patent Application 2009/0240090. In a practice of this embodiment, the invention is directed to a multistep process for preparing 2,3,3,3-tetrafluoropropene (1234yf) comprising: a.) vaporizing a starting composition comprising at least one compound of formula I

CX₂=CCl-CH₂X (I)

wherein X is independently selected from F, CI, Br, and I, provided that at least one X is not fluorine, wherein the at least one compound of formula I comprises 1,1,2,3-tetrachloropropene; and a fluorinating agent and one or more organic co-feed compounds selected from the group consisting of dichlorodifluoropropene (1232), 1,2-dichloro-3,3,3-trifluoropropene (1223xd), 2-chloro-3,3,3-trifluoropropene (1233xf), 3,3,3,2-tetrafluoroprop-1-ene (1234yf), 2-chloro-1,1,1,2-tetrafluoropropane (244bb), 1,1,1,2,2-pentafluoropropane (HFC-245cb), 1,1,1,2,3-pentafluoropropane (245eb), tetrachlorofluoropropane (241), trichlorodifluoropropane (242), dichlorotrifluoropropane (243) and combinations thereof, said at least one or more organic co-feed compound being present in an amount ranging from 1 to 50 wt %, preferably from 3 to 30 wt % and more preferably from 5 to 15 wt %; and contacting the vaporized compound of formula I and fluorinating agent with a fluorination catalyst to produce a first intermediate composition comprising 2-chloro-3,3,3-trifluoropropene (1233xf), HCl and one or more organic co-feed compounds, said one or more organic co-feed compounds being other than the compound of formula I;
b.) separating said HCl, said 2-chloro-3,3,3-trifluoropropene (1233xf), and said one or more organic co-feed compounds from said first intermediate composition; c.) recycling an effective amount of said separated one or more organic co-feed compounds to said first vapor phase reactor; d.) contacting, in a liquid phase reactor, said separated 2-chloro-3,3,3-trifluoropropene (1233xf) with a second fluorinating agent to produce a second intermediate composition comprising 2-chloro-1,1,1,2-tetrafluoropropane (244bb); and e.) dehydrochlorinating, in a second vapor phase reactor, at least a portion of said 2-chloro-1,1,1,2-tetrafluoropropane (244bb) to produce a reaction product comprising 2,3,3,3-tetrafluoropropene. In another practice of this embodiment, the recycling of said separated one or more organic co-feed compounds to said first vapor phase reactor in step c) provides said vapor phase catalyst with a longer catalyst life than in the absence of said recycling. In another practice of this embodiment, the vapor phase catalyst is selected from chromium oxide (Cr₂O₃), FeCl₃/C, Cr₂O₃/Al₂O₃, Cr₂O₃/AlF₃, Cr₂O₃/carbon, CoCl_{2/}Cr₂O₃/Al₂O₃, NiCl₂/Cr₂O₃/Al₂O₃, CoCl₂/AlF₃, NiCl₂/AlF₃ and mixtures thereof. In another practice of this embodiment, 244bb and/or 245cb are absent from the one or more organic co-feed compounds recycled to the first vapor phase reactor in step c), or alternatively, 244bb and/or 245cb are present in the recycle of step c) in insignificant amounts, e.g. the recycle is substantially free of 244bb and/or 245cb, which includes without limitation the presence of 244bb and/or 245cb in amounts that are not effective amounts. In another embodiment, the organic co-feeds can be a combination of fresh feed and recycle.

Optionally, the reaction may also include the use of one or more stabilizers. Generally speaking, such compounds may include an amine-based stabilizer. Non-limiting examples of such stabilizers suitable for use in the present reaction include those known for use in halogenation reactions, and in particular halogenation reactions involving alkanes, alkenes, and alkynes. In some embodiments, the stabilizer is selected from the group comprising p-tap (4-tert-amylphenol), methoxy-hydroquinone, 4-methoxyphenol (HQMME), triethylamine, di-isopropyl amine, butylated hydroxy anisole (BHA), thymol and combinations thereof. In certain preferred embodiments, the stabilizer comprises an amine-based stabilizer. More preferably, the stabilizer comprises triethylamine, di-isopropyl amine or combinations thereof. The stabilizer is preferably present in an amount less than 300 ppm, more preferably in an amount less than 100 ppm, and most preferably, in an amount less than 10 ppm.

The HF, compound of formula I, and organic co-feed may be fed continuously to a vaporizer and the vaporized reactants to the catalyst bed. When the compound of formula I is 1230xa, the mol ratio of HF to 1230xa in step 1 of the reaction is 1:1 to 50:1 and, in certain embodiments, from about 10:1 to about 20:1. The reaction between HF and 1230xa is carried out at a temperature from about 150°C to about 400°C (in certain embodiments, about 180°C to about 300°C) and at a pressure of about 0 psig to about 200 psig (0 to 2000 kPag) (in certain embodiments from about 0 psig to about 100 psig [0 to 1000 kPag]). Contact time of the 1230xa with the catalyst may range from about 1 second to about 60 seconds, however, longer or shorter times can be used.

The fluorination reaction is preferably carried out to attain a conversion of about 50% or, preferably, about 90% or higher. Conversion is calculated by the number of moles of reactant (1230xa) consumed divided by number of moles of reactant (1230xa) fed to the reactor multiplied by 100. The selectivity for 1233xf attained is preferably about 60% or higher and more preferably about 80% or higher. Selectivity is calculated by number of moles of product (1233xf) formed divided by number of moles of reactant consumed.

This first step of the reaction may be conducted in any reactor suitable for a vapor phase fluorination reaction. In certain embodiments, the reactor is constructed from materials which are resistant to the corrosive effects of hydrogen fluoride and catalyst such as Hastalloy, Nickel, Incoloy, Inconel, Monel and fluoropolymer linings. The vessel is a fixed catalyst bed or fluidized bed. If desired, inert gases such as nitrogen or argon may be employed in the reactor during operation.

In general, the effluent from the fluorination reaction step, including any intermediate effluents that may be present in multi-stage reactor arrangements, may be processed to achieve desired degrees of separation and/or other processing. For example, in embodiments in which the reactor effluent includes 1233xf, the effluent will generally also include HCl and one or more of HF, dichlorodifluoropropenes (1232), 1,2-dichloro-3,3,3-trifluoropropene (1223xd), trichlorofluoropropene (1231) isomers, 2-chloro-1,1,1,2-tetrafluoropropane (244bb), and unreacted 1230xa. Some portion or substantially all of these components of the reaction product may be recovered from the reaction mixture via any separation or purification method known in the art such as neutralization and distillation. It is expected that unreacted 1230xa and HF could be recycled, completely or partially, to improve the overall yield of the desired 1233xf. 1232 and any 1231 formed may also be recycled.

Optionally, hydrogen chloride is then recovered from the result of the fluorination reaction. Recovering of hydrogen chloride is conducted by conventional distillation where it is removed from the distillate. Alternatively, HCl can be recovered or removed by using water or caustic scrubbers. When a water extractor is used, HCl is removed as an aqueous solution. When caustic scrubbers are used, HCl is just removed from system as a chloride salt in aqueous solution.

In the second step of the process for forming 2,3,3,3-tetrafluoroprop-1-ene, 1233xf is converted to 2-chloro-1,1,1,2-tetrafluoropropane (244bb). In one embodiment, this step may be performed in the liquid phase in a liquid phase reactor, which may be TFE or PFA-lined. Such a process may be performed in a temperature range of about 70-120 °C and about 50-120 psig (300 to 830 kPag).

Any liquid phase fluorination catalyst may be used in the invention. A non-exhaustive list include Lewis acids, transition metal halides, transition metal oxides, Group IVb metal halides, a Group Vb metal halides, or combinations thereof. Non-exclusive examples of liquid phase fluorination catalysts are an antimony halide, a tin halide, a tantalum halide, a titanium halide, a niobium halide, and molybdenum halide, an iron halide, a fluorinated chrome halide, a fluorinated chrome oxide or combinations thereof. Specific non-exclusive examples of liquid phase fluorination catalysts are SbCl₅, SbCl₃, SbF₅, SnCl₄, TaCl₅, TiCl₄, NbCl₅, MoCl₆, FeCl₃, a fluorinated species of SbCl₅, a fluorinated species of SbCl₃, a fluorinated species of SnCl₄, a fluorinated species of TaCl₅, a fluorinated species of TiCl₄, a fluorinated species of NbCl₅, a fluorinated species of MoCl₆, a fluorinated species of FeCl₃, or combinations thereof. Antimony pentachloride is most preferred.

These catalysts can be readily regenerated by any means known in the art if they become deactivated. One suitable method of regenerating the catalyst involves flowing a stream of chlorine through the catalyst. For example, from about 0.002 to about 0.2 lb per hour (0.9 to 90 g/h) of chlorine can be added to the liquid phase reaction for every pound (450 g) of liquid phase fluorination catalyst. This may be done, for example, for from about 1 to about 2 hours or continuously at a temperature of from about 65 °C to about 100 °C.

This second step of the reaction is not necessarily limited to a liquid phase reaction and may also be performed using a vapor phase reaction or a combination of liquid and vapor phases, such as that disclosed in U.S. Published Patent Application No. 20070197842. To this end, the 1233xf containing feed stream is preheated to a temperature of from about 50 °C to about 400 °C, and is contacted with a catalyst and fluorinating agent. Catalysts may include standard vapor phase agents used for such a reaction and fluorinating agents may include those generally known in the art, such as, but not limited to, hydrogen fluoride.

In the third step of 1234yf production, the 244bb is fed to a second vapor phase reactor (dehydrochlorination reactor) to be dehydrochlorinated to make the desired product 2,3,3,3-tetrafluoroprop-1-ene (1234yf). This reactor contains a catalyst that can catalytically dehydrochlorinate HCFC-244bb to make HFO-1234yf.

The catalysts may be metal halides, halogenated metal oxides, neutral (or zero oxidation state) metal or metal alloy, or activated carbon in bulk or supported form. Metal halide or metal oxide catalysts may include, but are not limited to, mono-, bi-, and tri-valent metal halides, oxides and their mixtures/combinations, and more preferably mono-, and bi-valent metal halides and their mixtures/combinations. Component metals include, but are not limited to, Cr³⁺, Fe³⁺, Mg²⁺, Ca²⁺, Ni²⁺, Zn²⁺, Pd²⁺, Li⁺, Na⁺, K⁺, and Cs⁺. Component halogens include, but are not limited to, F⁻, Cl⁻, Br⁻, and I⁻. Examples of useful mono- or bi-valent metal halide include, but are not limited to, LiF, NaF, KF, CsF, MgF₂, CaF₂, LiCl, NaCl, KCl, and CsCl. Halogenation treatments can include any of those known in the prior art, particularly those that employ HF, F₂, HCl, Cl₂, HBr, Br₂, HI, and I₂ as the halogenation source.

When neutral, i.e., zero valent, metals, metal alloys and their mixtures are used. Useful metals include, but are not limited to, Pd, Pt, Rh, Fe, Co, Ni, Cu, Mo, Cr, Mn, and combinations of the foregoing as alloys or mixtures. The catalyst may be supported or unsupported. Useful examples of metal alloys include, but are not limited to, SS 316, Monel 400, Inconel 825, Inconel 600, and Inconel 625.

Preferred, but non-limiting, catalysts include activated carbon, stainless steel (e.g. SS 316), austenitic nickel-based alloys (e.g. Inconel 625), nickel, fluorinated 10% CsCl/MgO, and 10% CsCl/MgF. The reaction temperature is preferably about 300-550 °C and the reaction pressure may be between about 0-150 psig (0 to 1000 kPag). The reactor effluent may be fed to a caustic scrubber or to a distillation column to remove the by-product of HCl to produce an acid-free organic product which, optionally, may undergo further purification using one or any combination of purification techniques that are known in the art.

The following are examples of the invention and are not to be construed as limiting.

### EXAMPLES

### Example 1: Fluorination of 1230xa using 1230xa as feed stock

3cc high surface area BASF chromium oxide was loaded into a 1/2 inch (1.3 cm) Hastelloy C 276 reactor. 6 inch (15 cm) Hastelloy B 1/8" (0.3 cm) distillation packing was packed on top of the catalyst as vaporizing zone. The catalyst was activated by HF first, then 1230xa was fed from top of the reactor at rate of 0.54 ml/hr together with 18 sccm HF and 3 sccm N₂ at 275 °C at atmospheric pressure. The stream from the reactor was analyzed by GC and GC-MS. The result of the test is shown in Figure 1. The catalyst shows degradation over the time.

### Example 2: Fluorination of 1230xa using 1232xf -1230xa (1:1 mol ratio) mixture as feed stock

3cc high surface area BASF chromium oxide was loaded into a 1/2 inch (1.3 cm) Hastelloy C 276 reactor. 6 inch (15 cm) Hastelloy B 1/8" (0.3 cm) distillation packing was packed on top of the catalyst as vaporizing zone. The catalyst was activated by HF first, then 1232xf-1230xa mixture was fed from top of the reactor at rate of 0.53 ml/hr together with 18 sccm HF and 3 sccm N₂ at 275 °C at atmosphere pressure. The stream from the reactor was analyzed by GC and GC-MS. The result of the test is shown in Figure 1. It shows improved catalyst life by using 1232xf -1230xa (1:1 mol ratio) mixture as feed stock.

### Example 3: Fluorination of 1230xa using 10wt%1233xf-90wt%1230xa mixture as feedstock

The 1230xa used in this example contained 5 ppm di-isopropyl amine. A mixture of 10wt%1233xf-90wt%1230xa was made as feedstock. 6.5 L of pre-fluorinated chromium oxide catalyst was loaded into a 4 inch (10 cm) Monel 400 reactor. The reactor was heated up to about 180°C in nitrogen flow. Anhydrous HF feed was then started at a flow rate of 1.9 lb/h (860 g/h). After passing though a Mol Sieve 3A column at a flow rate of 1.1 lb/h (500 g/h), organic feed was combined with HF feed. The mixed HF and organic feed was introduced to a vaporizer for vaporization and then to the reactor for reaction. The reaction temperature (hot spot temperature) was increased to about 200°C once the reaction was initiated. The reactor pressure was set at 70 psig (480 kPag). Samples were periodically taken from the product stream and were analyzed by GC and GC-MS during reaction. The results showed that 1230xa conversion was almost 100% and the average selectivities to 1233xf, 1232xf, 244bb were about 97.9%, 0.3%, and 1.5%, respectively, during the period of time of the reaction study that lasted for about 300 hours.

3cc high surface area BASF chromium oxide was loaded into a 1/2 inch Hastelloy C 276 reactor. 6 inched Hastelloy B 1/8" distillation packing was packed on top of the catalyst as vaporizing zone. The catalyst was activated by HF first, then 1232xf-1230xa mixture was fed from top of the reactor at rate of 0.53 ml/hr together with 18sccm HF and 3 sccm N₂ at 275 °C at atmosphere pressure. The stream from the reactor was analyzed by GC and GC-MS. The result of the test is shown in Figure 1. It shows improved catalyst life by using 1232xf -1230xa (1:1 mol ratio) mixture as feed stock.

### Example 3: Fluorination of 1230xa using 10wt%1233xf-90wt%1230xa mixture as feedstock

The 1230xa used in this example contained 5 ppm di-isopropyl amine. A mixture of 10wt%1233xf-90wt%1230xa was made as feedstock. 6.5 L of pre-fluorinated chromium oxide catalyst was loaded into a 4 inch Monel 400 reactor. The reactor was heated up to about 180°C in nitrogen flow. Anhydrous HF feed was then started at a flow rate of 1.9 lb/h. After passing though a Mol Sieve 3A column at a flow rate of 1.1 lb/h, organic feed was combined with HF feed. The mixed HF and organic feed was introduced to a vaporizer for vaporization and then to the reactor for reaction. The reaction temperature (hot spot temperature) was increased to about 200°C once the reaction was initiated. The reactor pressure was set at 70 psig. Samples were periodically taken from the product stream and were analyzed by GC and GC-MS during reaction. The results showed that 1230xa conversion was almost 100% and the average selectivities to 1233xf, 1232xf, 244bb were about 97.9%, 0.3%, and 1.5%, respectively, during the period of time of the reaction study that lasted for about 300 hours.

## Claims

1. A process for preparing 2-chloro-3,3,3-trifluoropropene comprising:
vaporizing a fluorination agent and a composition comprising at least one compound of formula I
CX₂=CCI-CH₂X (I)
wherein X is independently selected from F, Cl, Br, and I, provided that at least one X is not fluorine, wherein the at least one compound of formula I comprises 1,1,2,3-tetrachloropropene; and one or more organic co-feed compounds, wherein said one or more organic co-feed compounds are selected from the group consisting of dichlorodifluoropropene (1232), 1,2-dichloro-3,3,3-trifluoropropene (1223xd), 2-chloro-3,3,3-trifluoropropene (1233xf), 3,3,3,2-tetrafluoroprop-1-ene (1234yf), 2-chloro-1,1,1,2-tetrafluoropropane (244bb), 1,1,1,2,2-pentafluoropropane (HFC-245cb), 1,1,1,2,3-pentafluoropropane (245eb), tetrachlorofluoropropane (241), trichlorodifluoropropane (242), dichlorotrifluoropropane (243) and combinations thereof, said one or more organic co-feed compounds being present in an amount ranging from 1 to 50 wt %, and preferably from 3 to 30 wt% and more preferably from 5 to 15 wt%; and
contacting the vaporized compound of formula I and the vaporized fluorinating agent in the vapor phase in the presence of a fluorination catalyst, to produce a final composition comprising 2-chloro-3,3,3-trifluoropropene.

2. The process of claim 1, wherein the organic co-feed is selected from the group consisting of dichlorodifluoropropene (1232), 2-chloro-3,3,3-trifluoropropene (1233xf) and combinations thereof.

3. The process of claim 1 or claim 2, wherein the fluorination catalyst is selected from the group consisting of a chromium oxide, a chromium hydroxide, a chromium halide, a chromium oxyhalide, an aluminum oxide, an aluminum hydroxide, an aluminum halide, an aluminum oxyhalide, a cobalt oxide, a cobalt hydroxide, a cobalt halide, a cobalt oxyhalide, a manganese oxide, a manganese hydroxide, a manganese halide, a manganese oxyhalide, a nickel oxide, a nickel hydroxide, a nickel halide, a nickel oxyhalide, an iron oxide, an iron hydroxide, an iron halide, an iron oxyhalide, inorganic salts thereof, fluorinated derivatives thereof and combinations thereof, more preferably wherein the catalyst comprises a chromium oxide, and even more preferably wherein the catalyst comprises Cr₂O₃.

4. The process of any preceding claim, wherein the contacting step occurs in the presence of at least one stabilizer.

5. The process of claim 4, wherein the stabilizer is an amine-based stabilizer.

6. The process of claim 5, wherein the stabilizer is selected from the group consisting of p-tap (4-tert-amylphenol), methoxy-hydroquinone, 4-methoxyphenol (HQMME), triethylamine, di-isopropyl amine, butylated hydroxyl anisole (BHA), thymol and combinations thereof.

7. The process of claim 6, wherein the stabilizer is triethylamine, di-isopropyl amine or combinations thereof.

8. The process of any of claims 5 to 7, wherein the stabilizer is present in an amount of less than 300 ppm.

9. A process for preparing 2,3,3,3-tetrafluoroprop-1-ene comprising:
a. vaporizing a composition comprising at least one compound of formula I
CX₂=CCl-CH₂X (I)
wherein X is independently selected from F, Cl, Br, and I, provided that at least one X is not fluorine, wherein the at least one compound of formula I comprises 1,1,2,3-tetrachloropropene; a first fluorinating agent and one or more organic co-feed compounds, wherein said one or more organic co-feed compounds is selected from the group consisting of dichlorodifluoropropene (1232), 1,2-dichloro-3,3,3-trifluoropropene (1223xd), 2-chloro-3,3,3-trifluoropropene (1233xf), 3,3,3,2-tetrafluoroprop-1-ene (1234yf), 2-chloro-1,1,1,2-tetrafluoropropane (244bb), 1,1,1,2,2-pentafluoropropane (HFC-245cb), 1,1,1,2,3-pentafluoropropane (245eb), tetrachlorofluoropropane (241), trichlorodifluoropropane (242), dichlorotrifluoropropane (243) and combinations thereof, said one or more organic co-feed compounds being present in an amount ranging from 1 to 50 wt %, preferably from 3 to 30 wt % and more preferably from 5 to 15 wt %;
b. contacting said vaporized compound of formula I and said vaporized first fluorinating agent with a fluorination catalyst to produce a first intermediate composition comprising 2-chloro-3,3,3-trifluoropropene;
c. contacting said first intermediate composition with a second fluorinating agent to produce a second intermediate composition comprising 2-chloro-1,1,1,2-tetrafluoropropane; and
d. dehydrochlorinating at least a portion of said 2-chloro-1,1,1,2-tetrafluoropropane to produce a reaction product comprising 2,3,3,3-tetrafluoroprop-1-ene.

10. A multistep process for preparing 2,3,3,3-tetrafluoropropene (1234yf) comprising:
a.) vaporizing a starting composition comprising at least one compound of formula I
CX₂=CCI-CH₂X (I)
wherein X is independently selected from F, Cl, Br, and I, provided that at least one X is not fluorine, wherein the at least one compound of formula I comprises 1,1,2,3-tetrachloropropene; and a fluorinating agent and one or more organic co-feed compounds selected from the group consisting of dichlorodifluoropropene (1232), 1,2-dichloro-3,3,3-trifluoropropene (1223xd), 2-chloro-3,3,3-trifluoropropene (1233xf), 3,3,3,2-tetrafluoroprop-1-ene (1234yf), 2-chloro-1,1,1,2-tetrafluoropropane (244bb), 1,1,1,2,2-pentafluoropropane (HFC-245cb), 1,1,1,2,3-pentafluoropropane (245eb), tetrachlorofluoropropane (241), trichlorodifluoropropane (242), dichlorotrifluoropropane (243) and combinations thereof, said one or more organic co-feed compounds being present in an amount ranging from 1 to 50 wt %, preferably from 3 to 30 wt % and more preferably from 5 to 15 wt%, and contacting the vaporized compound of formula I and fluorinating agent with a fluorination catalyst to produce a first intermediate composition comprising 2-chloro-3,3,3-trifluoropropene (1233xf), HCI and one or more organic co-feed compounds, said one or more organic co-feed compounds being other than the compound of formula I;
b.) separating said HCl, said 2-chloro-3,3,3-trifluoropropene (1233xf), and said one or more organic co-feed compounds from said first intermediate composition;
c.) recycling an effective amount of said separated one or more organic co-feed compounds to said first vapor phase reactor;
d.) contacting, in a liquid phase reactor, said separated 2-chloro-3,3,3-trifluoropropene (1233xf) with a fluorinating agent to produce a second intermediate composition comprising 2-chloro-1,1,1,2-tetrafluoropropane (244bb); and
e.) dehydrochlorinating, in a second vapor phase reactor, at least a portion of said 2-chloro-1,1,1,2-tetrafluoropropane (244bb) to produce a reaction product comprising 2,3,3,3-tetrafluoropropene.

11. The process of Claim 10 wherein said vapor phase catalyst is selected from Cr₂O₃, FeCl₃/C, Cr₂O₃/Al₂O₃, Cr₂O₃/AlF₃, Cr₂O₃/carbon, COCl₂/Cr₂O_{3/}Al₂O₃, NiCl₂/Cr₂O_{3/}Al₂O₃, CoCl₂/AlF₃, NiCl₂/AlF₃ and mixtures thereof.

12. The process of Claim 10 or 11 wherein said effective amount of said separated one or more organic co-feed compounds recycled to said first vapor phase reactor in step c) is between about 1 to about 50 wt% based on the total weight of said starting composition in step a).

13. A process for reducing oligimerization and polymerization in the process of preparing 2-chloro-3,3,3-trifluoropropene comprising vaporizing a composition comprising at least one compound of formula I
CX₂=CCI-CH₂X (I)
wherein X is independently selected from F, Cl, Br, and I, provided that at least one X is not fluorine, wherein the at least one compound of formula I comprises 1,1,2,3-tetrachloropropene; and a fluorinating agent in the presence of one or more organic co-feed compounds selected from the group consisting of dichlorodifluoropropene (1232), 1,2-dichloro-3,3,3-trifluoropropene (1223xd), 2-chloro-3,3,3-trifluoropropene (1233xf), 3,3,3,2-tetrafluoroprop-1-ene (1234yf), 2-chloro-1,1,1,2-tetrafluoropropane (244bb), 1,1,1,2,2-pentafluoropropane (HFC-245cb), 1,1,1,2,3-pentafluoropropane (245eb), tetrachlorofluoropropane (241), trichlorodifluoropropane (242), dichlorotrifluoropropane (243) and combinations thereof, said one or more organic co-feed compounds being present in an amount ranging from 1 to 50 wt %, preferably from 3 to 30 wt %, more preferably from 5 to 15 wt%; and
contacting said vaporized composition of Formula I and the vaporized fluorinating agent I in the vapor phase in the presence of a fluorination catalyst to produce a composition comprising 2-chloro-3,3,3-trifluoropropene.

14. The process of any of claims 9 to 13, wherein the organic co-feed is selected from the group consisting of dichlorodifluoropropene (1232), 2-chloro-3,3,3-trifluoropropene (1233xf) and combinations thereof.

## Patentansprüche

1. Verfahren zur Herstellung von 2-Chlor-3,3,3-trifluorpropen, umfassend:
- Verdampfen eines Fluorierungsmittels und einer Zusammensetzung, die mindestens eine Verbindung der Formel I
CX₂=CCI-CH₂X (I),
- worin X unabhängig aus F, CI, Br und I ausgewählt ist, mit der Maßgabe, dass mindestens ein X nicht Fluor ist, wobei die mindestens eine Verbindung der Formel I 1,1,2,3-Tetrachlorpropen umfasst, und eine oder mehrere organische Co-Feed-Verbindungen umfasst, wobei die eine oder mehreren organischen Co-Feed-Verbindungen aus der aus Dichlordifluorpropen (1232), 1,2-Dichlor-3,3,3-trifluorpropen (1223xd), 2-Chlor-3,3,3-trifluorpropan (1233xf), 3,3,3,2-Tetrafluorprop-1-en (1234yf), 2-Chlor-1,1,1,2-tetrafluorpropen (244bb), 1,1,1,2,2-Pentafluorpropan (HFC-245cb), 1,1,1,2,3-Pentafluorpropan (245eb), Tetrachlorfluorpropan (241), Trichlordifluorpropan (242), Dichlortrifluorpropan (243) und Kombinationen davon bestehenden Gruppe ausgewählt sind, wobei die eine oder die mehreren organischen Co-Feed-Verbindungen in einer Menge im Bereich von 1 bis 50 Gew.-% und vorzugsweise von 3 bis 30 Gew.-% und noch bevorzugter von 5 bis 15 Gew.-% vorhanden sind, und
- Inkontaktbringen der verdampften Verbindung der Formel I und des verdampften Fluorierungsmittels in der Dampfphase in Gegenwart eines Fluorierungskatalysators, um eine Endzusammensetzung herzustellen, die 2-Chlor-3,3,3-trifluorpropen enthält.

2. Verfahren nach Anspruch 1, wobei die organische Co-Feed-Verbindung aus der aus Dichlordifluorpropen (1232), 2-Chlor-3,3,3-trifluorpropen (1233xf) und Kombinationen davon bestehenden Gruppe ausgewählt ist.

3. Verfahren nach Anspruch 1 oder 2, wobei der Fluorierungskatalysator aus der aus einem Chromoxid, einem Chromhydroxid, einem Chromhalogenid, einem Chromoxyhalogenid, einem Aluminiumoxid, einem Aluminiumhydroxid, einem Aluminiumhalogenid, einem Aluminiumoxyhalogenid, einem Kobaltoxid, einem Kobalthydroxid, einem Kobalthalogenid, einem Kobaltoxyhalogenid, einem Manganoxid, einem Manganhydroxid, einem Manganhalogenid, einem Manganoxyhalogenid, einem Nickeloxid, einem Nickelhydroxid, einem Nickelhalogenid, einem Nickeloxyhalogenid, einem Eisenoxid, einem Eisenhydroxid, einem Eisenhalogenid, einem Eisenoxyhalogenid, anorganische Salze hiervon, fluorierte Derivate hiervon und Kombinationen hiervon bestehenden Gruppe ausgewählt ist, wobei der Katalysator vorzugsweise ein Chromoxid und, noch bevorzugter, wobei der Katalysator Cr₂O₃ umfasst.

4. Verfahren nach einem der vorhergehenden Ansprüche, wobei der Kontaktierungsschritt in Gegenwart mindestens eines Stabilisators erfolgt.

5. Verfahren nach Anspruch 4, wobei der Stabilisator ein Stabilisator auf Aminbasis ist.

6. Verfahren nach Anspruch 5, wobei der Stabilisator aus der aus p-tap (4-tert-Amylphenol), Methoxyhydrochinon, 4-Methoxyphenol (HQMME), Triethylamin, Diisopropylamin, butyliertem Hydroxylanisol (BHA), Thymol und Kombinationen davon bestehenden Gruppe ausgewählt ist.

7. Verfahren nach Anspruch 6, wobei der Stabilisator Triethylamin, Diisopropylamin oder Kombinationen davon ist.

8. Verfahren nach einem der Ansprüche 5 bis 7, wobei der Stabilisator in einer Menge von weniger als 300 ppm vorliegt.

9. Verfahren zur Herstellung von 2,3,3,3-Tetrafluoroprop-1-en, umfassend
a. Verdampfen einer Zusammensetzung, die mindestens eine Verbindung der Formel I
CX₂=CCI-CH₂X (I),
worin X unabhängig aus F, CI, Br und I ausgewählt ist, mit der Maßgabe, dass mindestens ein X nicht Fluor ist, wobei die mindestens eine Verbindung der Formel I 1,1,2,3-Tetrachlorpropen umfasst, ein erstes Fluorierungsmittel und eine oder mehrere organische Co-Feed-Verbindungen umfasst, wobei die eine oder mehreren organischen Co-Feed-Verbindungen aus der aus Dichlordifluorpropen (1232), 1,2-Dichlor-3,3,3-trifluorpropen (1223xd), 2-Chlor-3,3,3-trifluorpropen (1233xf), 3,3,3,2-Tetrafluorprop-1-en (1234yf), 2-Chlor-1,1,1,2-tetrafluorpropan (244bb), 1,1,1,2,2-Pentafluorpropan (HFC-245cb), 1,1,1,2,3-Pentafluorpropan (245eb), Tetrachlorfluorpropan (241), Trichlordifluorpropan (242), Dichlortrifluorpropan (243) und Kombinationen davon bestehenden Gruppe ausgewählt sind, wobei die eine oder die mehreren organischen Co-Feed-Verbindungen in einer Menge von 1 bis 50 Gew.-%, vorzugsweise von 3 bis 30 Gew.-% und besonders bevorzugt von 5 bis 15 Gew.-% vorhanden sind,
b. Inkontaktbringen der verdampften Verbindung der Formel I und des verdampften ersten Fluorierungsmittels mit einem Fluorierungskatalysator, um eine erste Zwischenproduktzusammensetzung herzustellen, die 2-Chlor-3,3,3-trifluorpropen enthält,
c. Inkontaktbringen der ersten Zwischenproduktzusammensetzung mit einem zweiten Fluorierungsmittel, um eine zweite Zwischenproduktzusammensetzung herzustellen, die 2-Chlor-1,1,1,2-tetrafluorpropan enthält, und
d. Dehydrochlorieren mindestens eines Teils des 2-Chlor-1,1,1,2-Tetrafluorpropans, um ein 2,3,3,3-Tetrafluorprop-1-en umfassendes Reaktionsprodukt herzustellen.

10. Mehrstufiges Verfahren zur Herstellung von 2,3,3,3-Tetrafluorpropen (1234yf) umfassend:
a.) Verdampfen einer Ausgangszusammensetzung, die mindestens eine Verbindung der Formel I
CX₂=CCI-CH₂X (I),
worin X unabhängig voneinander aus F, CI, Br und I ausgewählt ist, mit der Maßgabe, dass mindestens ein X nicht Fluor ist, wobei die mindestens eine Verbindung der Formel I 1,1,2,3-Tetrachlorpropen umfasst, und ein Fluorierungsmittel und eine oder mehrere organische Co-Feed-Verbindungen umfasst, ausgewählt aus der Gruppe bestehend aus Dichlordifluorpropen (1232), 1,2-Dichlor-3,3,3-Trifluorpropen (1223xd), 2-Chlor-3,3,3-Trifluorpropen (1233xf), 3,3,3,2-Tetrafluorprop-1-en(1234yf), 2-Chlor-1,1,1,2-tetrafluorpropan (244bb), 1,1,1,2,2-Pentafluorpropan (HFC-245cb), 1,1,1,2,3-Pentafluorpropan (245eb), Tetrachlorfluorpropan (241), Trichlordifluorpropan (242), Dichlortrifluorpropan (243) und Kombinationen davon, wobei die eine oder die mehreren organischen Co-Feed-Verbindungen in einer Menge im Bereich von 1 bis 50 Gew.-%, vorzugsweise von 3 bis 30 Gew.-% und besonders bevorzugt von 5 bis 15 Gew.-% vorhanden sind, und Inkontaktbringen der verdampften Verbindung der Formel I und des Fluorierungsmittels mit einem Fluorierungskatalysator, um eine erste Zwischenzusammensetzung zu erzeugen, die 2-Chlor-3,3,3-trifluorpropen (1233xf), HCI und eine oder mehrere organische Co-Feed-Verbindungen umfasst, wobei die eine oder mehreren organischen Co-Feed-Verbindungen von der Verbindung der Formel I verschieden sind,
b.) Abtrennen des HCI, des 2-Chlor-3,3,3-trifluoropropens (1233xf) und der einen oder mehreren organischen Co-Feed-Verbindungen aus der ersten Zwischenproduktzusammensetzung,
c.) Rückführung einer wirksamen Menge der abgetrennten einen oder mehreren organischen Co-Feed-Verbindungen zum ersten Dampfphasenreaktor,
d.) Inkontaktbringen des abgetrennten 2-Chlor-3,3,3-trifluorpropens (1233xf) mit einem Fluorierungsmittel in einem Flüssigphasenreaktor, um eine zweite Zwischenzusammensetzung herzustellen, die 2-Chlor-1,1,1,2-tetrafluorpropan (244bb) umfasst, und
e.) Dehydrochlorieren mindestens eines Teils des 2-Chlor-1,1,1,2-tetrafluorpropans (244bb) in einem zweiten Dampfphasenreaktor, um ein 2,3,3,3-Tetrafluorpropen umfassendes Reaktionsprodukt herzustellen.

11. Verfahren nach Anspruch 10, wobei der Dampfphasenkatalysator ausgewählt ist aus Cr₂O₃, FeCl₃/C, Cr₂O₃/Al₂O₃, Cr₂O₃/AlF₃, Cr₂O₃/Kohlenstoff, CoCl₂/Cr₂O₃/Al₂O₃, NiCl₂/Cr₂O₃/Al₂O₃, CoCl₂/AlF₃, NiCl₂/AlF₃ und Mischungen davon.

12. Verfahren nach Anspruch 10 oder 11, wobei die wirksame Menge der abgetrennten einen oder mehreren organischen Co-Feed-Verbindungen, die in den ersten Dampfphasenreaktor in Schritt c) zurückgeführt werden, zwischen etwa 1 und etwa 50 Gew.-%, bezogen auf das Gesamtgewicht der Ausgangszusammensetzung in Schritt a), beträgt.

13. Verfahren zur Verringerung der Oligomerisierung und Polymerisation im Verfahren zur Herstellung von 2-Chlor-3,3,3-trifluorpropen, umfassend das Verdampfen einer Zusammensetzung, die mindestens eine Verbindung der Formel I
CX₂=CCI-CH₂X (I),
- worin X unabhängig aus F, CI, Br und I ausgewählt ist, mit der Maßgabe, dass mindestens ein X nicht Fluor ist, wobei die mindestens eine Verbindung der Formel I 1,1,2,3-Tetrachlorpropen umfasst, und ein Fluorierungsmittel in Gegenwart einer oder mehrerer organischer Co-Feed-Verbindungen, ausgewählt aus der Gruppe bestehend aus Dichlordifluorpropen (1232), 1,2-Dichlor-3,3,3-trifluorpropen (1223xd), 2-Chlor-3,3,3-trifluorpropen (1233xf), 3,3,3,2-Tetrafluorprop-1-en (1234yf), 2-Chlor-1,1,1,2-tetrafluorpropan (244bb), 1,1,1,2,2-Pentafluorpropan (HFC-245cb), 1,1,1,2,3-Pentafluorpropan (245eb), Tetrachlorfluorpropan (241), Trichlordifluorpropan (242), Dichlortrifluorpropan (243) und Kombinationen davon, wobei die eine oder die mehreren organischen Co-Feed-Verbindungen in einer Menge im Bereich von 1 bis 50 Gew.-%, vorzugsweise von 3 bis 30 Gew.-%, noch bevorzugter von 5 bis 15 Gew.-% vorhanden sind, und
- Inkontaktbringen der verdampften Zusammensetzung der Formel I und des verdampften Fluorierungsmittels I in der Dampfphase in Gegenwart eines Fluorierungskatalysators, um eine 2-Chlor-3,3,3-trifluorpropen umfassende Zusammensetzung herzustellen.

14. Verfahren nach einem der Ansprüche 9 bis 13, wobei die organische Co-Feed-Verbindung aus der aus Dichlordifluorpropen (1232), 2-Chlor-3,3,3-trifluorpropen (1233xf) und Kombinationen davon bestehenden Gruppe ausgewählt ist.

## Revendications

1. Procédé de préparation du 2-chloro-3,3,3-trifluoropropène consistant à :
- vaporiser d'un agent de fluoration et d'une composition comprenant au moins un composé de formule I
CX₂=CCl-CH₂X (1)
dans laquelle
* X est indépendamment choisi parmi F, Cl, Br et I, à condition qu'au moins un X ne soit pas du fluor,
* le composé au moins de la formule I comprend 1,1,2,3-tétrachloropropène ; et un ou plusieurs composés organiques d'alimentation conjointe,
- ce ou ces composés organiques d'alimentation conjointe étant choisis dans le groupe comprenant : dichlorodifluoropropène (1232), 1,2-dichloro-3,3,3-trifluoropropène (1223xd), 2-chloro-3,3,3-trifluoropropène (1233xf), 3,3,3,2-tétrafluoroprop-1-ène (1234yf), 2-chloro-1,1,1,2-tétrafluoropropane (244bb), 1,1,1,2,2-pentafluoropropane (HFC-245cb), 1,1,1,2,3-pentafluoropropane (245eb), tétrachlorofluoropropane (241), trichlorodifluoropropane (242), dichlorotrifluoropropane (243) et leurs combinaisons,
- ce ou ces composés organiques d'alimentation étant présents en une quantité allant de 1 à 50 % en poids, et de préférence de 3 à 30 % en poids, et plus préférentiellement de 5 à 15 % en poids, et
- mettre en contact le composé de formule I vaporisé et de l'agent de fluoration vaporisé en phase vapeur en présence d'un catalyseur de fluoration, pour produire une composition finale comprenant du 2-chloro-3,3,3-trifluoropropène.

2. Procédé de la revendication 1,
selon lequel
- le co-aliment organique est choisi dans le groupe comprenant : dichlorodifluoropropène (1232), 2-chloro-3,3,3-trifluoropropène (1233xf) et leurs combinaisons.

3. Procédé de la revendication 1 ou 2,
selon lequel
le catalyseur de fluoration est choisi dans le groupe comprenant : oxyde de chrome, hydroxyde de chrome, halogénure de chrome, oxyhalogénure de chrome, oxyde d'aluminium, hydroxyde d'aluminium, halogénure d'aluminium, oxyhalogénure d'aluminium, oxyde de cobalt, hydroxyde de cobalt, halogénure de cobalt, oxyhalogénure de cobalt, oxyde de manganèse, hydroxyde de manganèse, halogénure de manganèse, oxyhalogénure de manganèse, oxyde de fer, hydroxyde de fer, halogénure de fer, oxyhalogénure de fer un halogénure de manganèse, oxyhalogénure de manganèse, oxyde de nickel, hydroxyde de nickel, halogénure de nickel, oxyhalogénure de nickel, oxyde de fer, hydroxyde de fer, halogénure de fer, oxyhalogénure de fer, leurs sels inorganiques, leurs dérivés fluorés et leurs combinaisons,
- plus préférentiellement le catalyseur comprend un oxyde de chrome, et encore plus préférentiellement le catalyseur comprend Cr₂O₃.

4. Procédé selon les revendications précédentes,
selon lequel
l'étape de mise en contact se fait en présence d'au moins un stabilisateur.

5. Procédé selon la revendication 4,
selon lequel
le stabilisant est à base d'amine.

6. Procédé selon la revendication 5,
selon lequel
- le stabilisant est choisi dans le groupe comprenant : p-tap (4-tert-amylphénol), méthoxy-hydroquinone, 4-méthoxyphénol (HQMME), triéthylamine, di-isopropylamine, butylhydroxylanisole (BHA), thymol et leurs combinaisons.

7. Procédé selon la revendication 6,
selon lequel
- le stabilisateur est la triéthylamine, la di-isopropylamine ou leurs combinaisons.

8. Procédé selon l'une des revendications 5 à 7,
selon lequel
- le stabilisant est présent en quantité inférieure à 300 ppm.

9. Procédé de préparation du 2,3,3,3-tetrafluoroprop-1-ène consistant à :
a) vaporiser une composition comprenant un composé de formule I
CX₂=CCl-CH₂ X (I)
dans laquelle
- X est indépendamment choisi parmi F, CI, Br et I, à condition qu'au moins un X ne soit pas du fluor,
- le composé de la formule I comprend du 1,1,2,3-tétrachloropropène ; un premier agent de fluoration et un ou plusieurs composés organiques d'alimentation conjointe, ce ou ces composés organiques d'alimentation conjointe étant choisis dans le groupe comprenant : dichlorodifluoropropène (1232), 1,2-dichloro-3,3,3-trifluoropropène (1223xd), 2-chloro-3,3,3-trifluoropropène (1233xf), 3,3,3,2-tetrafluoroprop-1-ène (1234yf), 2-chloro-1,1,1,2-tetrafluoropropane (244bb), 1,1,1,2,2-pentafluoropropane (HFC-245cb), 1,1,1,2,3-pentafluoropropane (245eb), tétrachlorofluoropropane (241), trichlorodifluoropropane (242), dichlorotrifluoropropane (243) et leurs combinaisons,
- ce ou ces composés organiques d'alimentation secondaire étant présents dans une proportion allant de 1 à 50 % en poids, de préférence de 3 à 30 % en poids et plus préférentiellement de 5 à 15 % en poids,
b) mettre en contact le composé de formule I vaporisé et le premier agent de fluoration vaporisé avec un catalyseur de fluoration pour produire une première composition intermédiaire comprenant du 2-chloro-3,3,3-trifluoropropène,
c) mettre en contact cette première composition intermédiaire avec un second agent de fluoration pour produire une seconde composition intermédiaire comprenant du 2-chloro-1,1,1,2-tétrafluoropropane, et
d) déshydrochlorer au moins une partie du 2-chloro-1,1,1,2-tétrafluoropropane pour obtenir un produit de réaction comprenant le 2,3,3,3-tétrafluoroprop-1-ène.

10. Procédé de préparation en plusieurs étapes du 2,3,3,3-tetrafluoropropène (1234yf) consistant à :
a) vaporiser une composition de départ comprenant un composé de formule I
CX₂=CCI-CH₂X (I)
dans laquelle
- où X est indépendamment choisi parmi F, CI, Br et I, à condition qu'au moins un X ne soit pas du fluor,
- le composé de la formule I comprend du 1,1,2,3-tétrachloropropène ; et un agent de fluoration et un ou plusieurs composés organiques d'alimentation complémentaire choisis dans le groupe comprenant : dichlorodifluoropropène (1232), 1,2-dichloro-3,3,3-trifluoropropène (1223xd), 2-chloro-3,3,3-trifluoropropène (1233xf), le 3,3,3,2-tétrafluoroprop-1-ène (1234yf), 2-chloro-1,1,1,2-tetrafluoropropane (244bb), 1,1,1,2,2-pentafluoropropane (HFC-245cb), 1,1,1,2,3-pentafluoropropane (245eb), tétrachlorofluoropropane (241), trichlorodifluoropropane (242), dichlorotrifluoropropane (243) et leurs combinaisons,
- ce ou ces composés organiques d'alimentation étant présents en une quantité allant de 1 à 50 % en poids, de préférence de 3 à 30 % en poids et de préférence encore de 5 à 15 % en poids, et
- mettre en contact le composé de formule I vaporisé et l'agent de fluoration avec un catalyseur de fluoration pour produire une première composition intermédiaire comprenant du 2-chloro-3,3,3-trifluoropropène (1233xf), du HCI et un ou plusieurs composés organiques d'alimentation,
- ce ou ces composés organiques d'alimentation étant autres que le composé de formule I,
b) séparer le HCI, le 2-chloro-3,3,3-trifluoropropène (1233xf) et ce ou ces composés organiques d'alimentation secondaire de la première composition intermédiaire,
c) recycler une quantité efficace de ces composés organiques séparés dans le premier réacteur en phase vapeur,
d) mettre en contact, dans un réacteur en phase liquide, le 2-chloro-3,3,3-trifluoropropène (1233xf) séparé avec un agent de fluoration pour produire une seconde composition intermédiaire comprenant du 2-chloro-1,1,1,2-tétrafluoropropane (244bb), et
e) déshydrochlorer, dans un second réacteur en phase vapeur, au moins une partie du 2-chloro-1,1,1,2-tétrafluoropropane (244bb) pour obtenir un produit de réaction comprenant le 2,3,3,3-tétrafluoropropène.

11. Procédé selon la revendication 10,
selon lequel
- le catalyseur en phase vapeur est choisi parmi Cr₂O₃, FeCl₃/C, Cr₂O₃/Al₂O₃, Cr₂O₃/AlF₃, Cr₂O₃/carbone, CpCl_{2/}Cr₂O₃/Al₂O₃, NiCl₂/Cr₂O₃/Al₂O₃, CoCl₂/AIF₃, NiCl₂/AIF₃ et leurs mélanges.

12. Procédé selon les revendications 10 ou 11,
selon lequel
- la quantité effective de ces composés organiques séparés recyclés dans le premier réacteur en phase vapeur à l'étape c) est comprise entre environ 1 et environ 50 % en poids par rapport au poids total de la composition de départ de l'étape a).

13. Procédé de réduction de l'oligimérisation et de la polymérisation du procédé de préparation du 2-chloro-3,3,3-trifluoropropène consistant à :
- vaporiser une composition comprenant au moins un composé de formule I.
CX₂=CCl-CH₂X (I)
dans laquelle
- X est choisi indépendamment parmi F, Cl, Br et I, à condition qu'au moins un X ne soit pas du fluor,
- le composé de la formule I comprenant du 1,1,2,3-tétrachloropropène ; et un agent de fluoration en présence d'un ou de plusieurs composés organiques cofiés choisis dans le groupe comprenant : dichlorodifluoropropène (1232), 1,2-dichloro-3,3,3-trifluoropropène (1223xd), 2-chloro-3,3,3-trifluoropropène (1233xf), 3,3,3,2-tétrafluoroprop-1-ène (1234yf), 2-chloro-1,1,1,2-tétrafluoropropane (244bb), 1,1,1,2,2-pentafluoropropane (HFC-245cb), 1,1,1,2,3-pentafluoropropane (245eb), tétrachlorofluoropropane (241), trichlorodifluoropropane (242), dichlorotrifluoropropane (243) et leurs combinaisons,
- le ou les composés organiques d'alimentation étant présents dans une proportion allant de 1 à 50 % en poids, de préférence de 3 à 30 % en poids, plus préférentiellement de 5 à 15 % en poids, et
- mettre en contact la composition vaporisée de formule I et l'agent de fluoration I vaporisé en phase vapeur en présence d'un catalyseur de fluoration pour produire une composition comprenant du 2-chloro-3,3,3-trifluoropropène.

14. Procédé selon l'une des revendications 9 à 13,
selon lequel
le co-aliment organique est choisi dans le groupe comprenant : dichlorodifluoropropène (1232), 2-chloro-3,3,3-trifluoropropène (1233xf) et leurs combinaisons.
